(19) Europäisches Patentamt
European Patent Office
Office européen des brevets

(11) Publication number: 0 518 441 A1

# (12) EUROPEAN PATENT APPLICATION

(21) Application number: 92201703.3

(51) Int. Cl.5: C07C 37/56, C07C 39/04

(22) Date of filing: 11.06.92

(30) Priority: 14.06.91 BE 9100579

(43) Date of publication of application:
16.12.92 Bulletin 92/51

(84) Designated Contracting States:
AT BE CH DE DK ES FR GB GR IT LI NL PT SE

(71) Applicant: DSM N.V.
Het Overloon 1
NL-6411 TE Heerlen(NL)

(72) Inventor: Buijs, Wim
Wolfhagen 145
NL-6365 BM Schinnen(NL)
Inventor: Offermanns, Matthias Robert Jozef
Koperslagerstraat 20
NL-6151 DK Sittard(NL)
Inventor: Frijns, Leon Hubertus Barbara
Cremerstraat 8
NL-6301 GE Valkenburg (L.)(NL)

## (54) Process for the preparation of a phenol.

(57) Process for the preparation of a phenol by an oxidative decarboxylation in the liquid phase of a corresponding arylcarboxylic acid in the presence of a Cu(I)-containing catalyst, characterized in that an oxidative decarboxylation is carried out at a temperature between 191-270 °C, in the presence of water, with such an amount of oxygen being added that so much copper remains present as Cu(I) that a selectivity to phenol is obtained of more than 92% measured at 1 bar, and phenol is separated off.

The oxidative decarboxylation is preferably carried out by:

a) oxidation of the catalyst at a temperature of 191-240 °C for at most 20 minutes, with such an amount of oxygen being added that at least 0.04 wt.% copper remains present as Cu(I);

b) reaction of the oxidized catalyst of step a) and formation of the phenol in the absence of oxygen, in the presence of water, at a temperature of 210-270 °C.

EP 0 518 441 A1

The invention relates to a process for the preparation of a phenol by an oxidative decarboxylation in the liquid phase of a corresponding arylcarboxylic acid in the presence of a Cu(I)-containing catalyst.

The preparation of a phenol by an oxidative decarboxylation has long been known. GB-A-762738, which is an equivalent of NL-B-90.684 already discloses such a process, wherein the oxidation, the decarboxylation as well as the reduction are effected in a single process step, at a temperature of at least 200 ° C, preferably 230-250 ° C.

In relation with this process a number of patent publications have appeared over the years, aiming to suppress the principal drawback of said process, which is the formation of a considerable number of by-products, mainly in the form of tar. In practice, between 15 and 25% tar is formed in continuous process operation.

In GB-A-1290626, which is an equivalent of NL-A-70.00685 a two-step process for the preparation of a phenol from a benzene monocarboxylic acid is described. First, the oxidation and the decarboxylation are carried out simultaneously at a temperature of 230-240 ° C. Next, hydrolysis of the resulting corresponding phenylbenzoate is effected in the presence of oxygen at a temperature of about 200 ° C.

GB-A-2001317, which is an equivalent of NL-A-78.07199 describes a process for the preparation of a phenol on the basis of a three-step process. First, an oxidation is effected in the absence of water, at a temperature of preferably 120-170 ° C; then a decarboxylation in the absence of oxygen and water, at a temperature preferably below 220 ° C, after which in a third step a hydrolysis of the resulting arylbenzoate takes place in the absence of oxygen, preferably at a temperature of about 220 ° C. According to the applicant for the patent in question, the presence of water in the decarboxylation should be avoided, which is achieved by addition of a dehydrating agent, by azeotropic distillation with an extra hydrocarbon added or by stripping with a dry inert gas.

However, all said processes have not enabled the formation of the phenol to be accomplished with a high yield under economically sound circumstances.

The process according to the invention provides a process for the preparation of a phenol from a corresponding arylcarboxylic acid with elimination of the drawbacks encountered in the above-mentioned processes. Thus a process is obtained which combines high preparation selectivities with an economically attractive embodiment. The process according to the invention is characterized in that the oxidative decarboxylation is carried out at a temperature between 191-270 ° C with such an amount of oxygen being added that so much copper remains present as Cu(I) that a selectivity to phenol is obtained of more than 92% measured at 1 bar, and phenol is separated off.

This process can simply be carried out continuously in one reaction vessel. For the sake of simplicity, the term 'one-step process' will be used in the following.

The one-step process is preferably carried out at 210-250 ° C. Although application of the inventions makes it possible to achieve a 10-20% higher selectivity than in the conventional process, some tar is still formed in the one-step process. Therefore it can be efficacious to remove part of the reactor mass and if desired free it of tar, after which copper and benzoic acid can be brought back into the process.

By keeping the Cu(I) concentration at a certain minimum value (see below), the yield improves to more than 92%, preferably more than 95% (if measured at 1 bar).

A virtually 100% selectivity is achieved if the following process steps are carried out:

a) oxidation of the catalyst at a temperature of 191-240 ° C, with such an amount of oxygen being added that at least 0.04 wt.% copper remains present as Cu(I);

b) reaction of the oxidized catalyst of step a) and formation of the phenol in the absence of oxygen, in the presence of water, at a temperature of 210-270 ° C;

c) separation of phenol and recycling of the (reduced) catalyst to step a).

By performing the process in this way, a two-step process is obtained: first an oxidation, then a reduction and formation of the phenol.

In this way the number of process steps is reduced in comparison with the process described in GB-A-2001317. In addition it appears that the process according to the invention gives a higher yield.

Here and below, arylcarboxylic acid is understood to be a compound having the following structure:

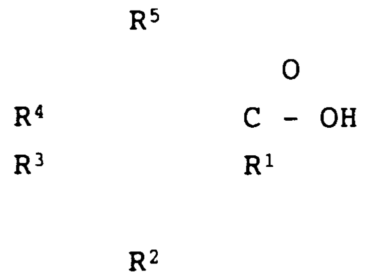

(I)

where $R^1$ through $R^5$ may be hydrogen (on the proviso that at least $R^1$ or $R^5$ is hydrogen) or organic groups, which have a so-called Hammett constant of between -1 and +2. A description of this Hammett or $\sigma$ value, which represents a measure of the influence of the group on the reactivity of the arylcarboxylic acid, can be found in J. March, Advanced Organic Chemistry 1989, pages 242-250; see in particular table 4 on page 244. The groups that can be used therefore are: $C_1$-$C_6$ alkyl, cylcoalkyl, aryl, arylalkyl, amino, halogen, nitro.

It is surprising that by the process according to the invention, metanitrobenzoic acid is converted into p-nitrophenol with a selectivity of >99% (the o-nitrophenol was only present in the form of traces).

Salts, esters and anhydrides of (I) are also suitable, while the groups may also be connected with each other via a ring system, as is the case for instance in naphthalene carboxylic acid (substituted or not). Multiple arylcarboxylic acids, such as trimellitic acid and pyromellitic acid, can also be used as starting material. Mixtures of the arylcarboxylic acids described above can also be used in the process according to the invention.

The invention relates particularly to a process for the conversion of unsubstituted benzoic acid ($R^1$ through $R^5$ = hydrogen) into the corresponding unsubstituted phenol.

The oxidation of the Cu-containing catalyst, involving conversion of Cu(I) arylcarboxylate into Cu(II) arylcarboxylate, is a first reaction step in the process. It brings about an increase in the degree of oxidation of the copper (from 1+ to 2+) as well as incorporation of an extra arylcarboxylate part into the Cu-containing catalyst.

In a one-step process the concentration of copper in the Cu(I) form preferably remains higher than 0.15 wt.% relative to the reaction mass, in particular higher than 0.2 wt.%. The difference with the two-step process in respect of the Cu(I) concentration to be preferably applied is mainly a consequence of the hydrodynamic behaviour of the reactor under these circumstances.

Surprisingly, it is found that formation of tar virtually does not occur.

The upper limit is not critical, but sufficient Cu(II) is advantageous in that it promotes a high productivity. Therefore the aim will be to have more than 50% copper oxidized to Cu(II).

The oxidation of Cu(I) to Cu(II) proceeds well particularly when carried out using an oxygen-containing gas. Air, whether or not enriched with oxygen or oxygen-depleted, can very well be used for this. Preferably, a gas is used with an oxygen content below the explosion limit. Depending on the medium, gas with an oxygen content of 1-15% can be used.

Such a gas can be passed through the Cu-containing liquid, for instance in a bubble-type washer. The pressure applied is not critical, but in general an elevated pressure will be chosen so as to accelerate the oxidation process. Pressures of 0.1-2.5 MPa are therefore suitable.

By continuous measurement of the gas flows it is easy to determine how much Cu(I) has been oxidized to Cu(II). On the one hand, the amount of oxygen taken up can be determined (by measuring the oxygen concentration in the incoming and outgoing gas flow), on the other the amount of Cu(I) in the catalyst added to the oxidation step. If the conversion into Cu(II) threatens to become too high, the gas supply can be shut off for example or inerts can be supplied for example. If the conversion is not high enough, for example more gas or for example a gas mixture with a higher oxygen concentration can be supplied to the reactor.

The amount of Cu-containing catalyst is to be chosen so that a good activity is obtained, but preferably it should not be so large as to give rise to the presence of a separate, solid catalyst phase throughout the process. The catalyst is in general dissolved in the reaction mixture. The copper concentration (as metal) in the oxidation step as a rule amounts to 0.5-15 wt.%, more preferably 1-10 wt.%; the process is best carried out with a copper concentration between 1.5 and 8 wt.%, in particular 3-6 wt.% (all relative to the reaction mixture in the oxidation step). Since in a process according to the present invention virtually no tar is formed, it is possible to use larger amounts of copper. The process according to GB-A-762738 is usually carried out with 1% copper, because separating off of tar, from a reactor mass with more than 1% copper is

problematic and because the copper losses become too high in that case, which is an economic drawback.

There may be advantage in using a catalyst containing a co-catalyst besides copper. This co-catalyst can be chosen in particular from groups V, VI, VII and VIII as well as from the group of the lanthanides and actinides of the Periodic System of the Elements. These components have an effect on the oxidative capacity of the Cu in the catalyst. Furthermore, promoters may be used, suitable materials being in particular (earth) alkaline metals, such as Mg or Li.

Preferably, these co-catalysts and/or promoters are used in a quantity of 1- 10 wt.%.

In the case of the two-step synthesis the process is preferably carried out under such conditions that the subsequent steps (viz. the reduction and formation of phenol) do not yet take place to any significant extent. At lower temperatures (e.g. 191-200 ° C) the first step may take up to 20 minutes, at higher temperatures a shorter liquid retention time will be chosen. Preferably, the liquid retention time is 1-10 minutes.

It is preferred in particular in the case of a two-step synthesis to oxidize the Cu-containing catalyst in accordance with the process described above at a temperature of 210-240 ° C.

The second reaction step in the process according to the invention comprises reduction of the catalyst and formation of the phenol, with release of carbon dioxide ($CO_2$).

In a one-step process the same process conditions will in general be applicable as described above for the first reaction step. As a rule, 0.3-5 wt.% water is present then, preferably 0.5-3 wt.%.

In the two-step process the second reaction step is preferably continued until 30-90% of the Cu(II) has been converted, in particular 60-90%.

In contrast to what is stated in GB-A-2001317, it has thus proved to be essential in the second step of the process according to the invention for water to be present in the reduction step. Further it has appeared that use of a temperature of 210-270 ° C, preferably a temperature of 220-250 ° C, results in an improvement of the selectivity.

In the two-step process both the reduction and the formation of phenol are effected in the absence of oxygen, in contrast to the process described in GB-A-1290626, referred to in the foregoing. Owing to the absence of oxygen, reoxidation of the Cu-containing catalyst in this process step is avoided, so that no (adverse) reactions can occur between the phenol or its intermediate products and any oxidized catalyst products. Such consecutive reactions appeared to give rise to the formation of selectivity-reducing by-products (such as tar).

Energetically, it is highly advantageous to carry out steps a) and b) at about the same temperature, preferably between 220-240 ° C.

Before supplying the reaction mixture from the first step to the second process step, it can be advantageous to remove part of the arylcarboxylic acid from the reaction mixture.

This can be done for example by means of partial evaporation (e.g. by distillation) of the reaction mixture obtained in the first step. Depending on its quality, the arylcarboxylic acid so obtained, can be sent on to the upgrading section (see below) or supplied directly to the oxidation step.

It is advantageous to use such an amount of water in the second step of the process according to the invention as to achieve virtual equimolarity relative to the amount of Cu(II). As a result, the reaction product obtained upon completion of the formation of phenol is virtually free of water, which is of advantage in the further upgrading to pure phenol of the reaction product thus obtained. But the amount of water may also be 2 to 3 times larger than the equimolar amount relative to Cu(II). The amount of water as a rule is 0.2-5 wt.%, preferably 0.5-4 wt.%, in particular 1-3 wt.%.

The pressure under which the second process step is performed is not critical, but the advantage of raising the pressure to above atmospheric level is that it has a favourable effect on the reaction kinetics and that the volatility of the reaction product is reduced. The pressure to be applied will generally be between 0.1 and 2.5 MPa; higher pressures, though allowable, do not yield substantial improvements of the process.

The second step as a rule takes 0.05-8 hours (depending on the temperature), preferably 0.1-3 hours.

A two-step process will as a rule be carried out as a continuous process, with the oxidation taking place in a first reaction vessel, and in a second one the reduction and formation of phenol. But the reaction can also be carried out in one vessel, batchwise and/or intermittently.

After the second step the reaction mixture is subjected to an upgrading operation to separate and recover the phenol obtained. This can be done in ways known by themselves, for instance by distillation. Certainly if hardly any water is left in the reaction mixture (in contrast to the process described in GB-A-762738, which is also referred to as the "wet route"), there is no longer any need to use an auxiliary material (such as toluene, to break up the phenol-water azeotrope) in the distillation. The bottom flow of the distillation, containing non-converted arylcarboxylic acid and the Cu-containing catalyst, can be recycled to the oxidation step, optionally after a purification step.

The process according to the invention is suitable in particular for the preparation of unsubstituted phenol from unsubstituted benzoic acid. This phenol can be used for instance as starting material both for phenol-formaldehyde resins and for the preparation of caprolactam, starting material for nylon-6, or for the preparation of bisphenol-A.

The invention will now be elucidated in the following examples, which should not be construed as limiting the invention.

## Examples

The following examples have all been conducted in an oil-heated, double-walled reactor having an effective volume of 0.5 l. This reactor was provided with a stirrer, gas inlet, steam inlet, gas outlet, distillation set-up, sampling point and reactor mass tapping point. For the comparative examples, use was made of a gas cylinder filled with a gas of a calibrated composition (nitrogen with 4.8 vol.% oxygen) to feed gas to the reactor. After cooling of the condensible products, the off-gas was analyzed for oxygen and carbon dioxide. In all experiments, performed batchwise, use was made of 350 g of reactor mass, consisting of the desired amounts of catalyst, co-catalyst and promoter, with arylcarboxylic acid added up to the total weight.

The catalyst, co-catalyst and promoter were supplied as the metal oxide or as the metal arylcarboxylate, with identical results in both cases.

The conversion of Cu(I) to Cu(II) in oxidation reactions was determined in two ways: a) by direct analysis of Cu(I), Cu(II) and total Cu in the reactor mass, and b) by drawing up a gas balance across the oxidation.

The conversion of Cu(II) to Cu(I) in the decomposition/hydrolysis reactions was also determined in two ways: a) by direct analysis of Cu(I), Cu(II) and total Cu in the reactor mass, and b) from the sum of all (auto)coupling products of an arylcarboxylic acid. These products were analyzed by HPLC. The conversions determined in this way were in good agreement (the deviation was less than 1%).

The results stated in the following description of the experiments were obtained as follows:

a) The yield of phenol ($\Sigma F$) is expressed as the total of moles of phenol + phenylbenzoate + p-phenylcarboxybenzoic acid + p-hydroxybenzoic acid obtained.

b) The yield of m-product ($\Sigma$m-product) is expressed as the total of moles of m-phenylcarboxybenzoic acid + m-hydroxybenzoic acid obtained (only relevant for experiments D-G).

c) The yield of total products ($\Sigma$ products) is expressed as $\Sigma F$ + $\Sigma$m-product + by-products.

d) the selectivity to phenol ($S_f$) is expressed as $\Sigma F/\Sigma$ products x 100%.

The mass, aromatic ring, $CO_2$ and $O_2$ balances had a value of 99 tot 101% for all examples.

## Examples I-II and comparative experiments A and B

A solution of Cu(I) benzoate in benzoic acid was oxidized to a certain degree. Then the solution of Cu-(II) benzoate thus obtained was decomposed and hydrolized at a suitable temperature. The resulting phenol was distilled off to about 1% (w/v) and the solution was filled up with fresh benzoic acid to the original weight. Then the solution was oxidized again, etc. The cycle was completed at least five times.
The results are given in table 1.

Table 1

| No. | oxidation Temp. (°C) | decomposition/ hydrolysis (°C) | (co-)cat (% metal) | $S_f$ | [Cu(I)] oxid. ended % (w/v) |
|---|---|---|---|---|---|
| I | 200 | 230 | 1% Cu, 3.5% Mg | 100% | 0.05% |
| A | 200 | 230 | 1% Cu, 3.5% Mg | 91% | <0.005% |
| II | 225 | 225 | 4% Cu, 2% Mg | 100% | 0.07% |
| B | 225 | 225 | 4% Cu, 2% Mg | 88% | <0.005% |

## Examples IV-V and comparative experiment C

A solution as described above was simultaneously oxidized, decomposed and hydrolyzed. The resulting phenol was continuously distilled off to about 1% (w/v). The solution was filled up with fresh arylcarboxylic

acid to the original weight. The experiment was continued for at least 7 hours.
The results are given in table 2.

Table 2

| No. | reactor mass T (°C) | (co-)cat (M%) (% metal) | $S_f$ | [Cu(I)], average (% metal) |
|---|---|---|---|---|
| C | 242 | 1% Cu, 3.5% Mg | 88% | 0.11% |
| III | 242 | 2% Cu, 3.5% Mg | 92% | 0.18% |
| IV | 242 | 3% Cu, 3.5% Mg | 97% | 0.24% |
| V[a)] | 230 | 4% Cu, 2% Mg | 98% | 2.12% |

[a)] Also strongly reduced gas load relative to experiments C, III and IV.

Comparative experiment D

Example I of GB-A-2001317 (NL-A-78.07199) was duplicated at a temperature of 218°C for 1 hour. The Cu(II) concentration required for a homogeneous catalyst application was 4.0 mol%.

Comparative experiment E

Experiment A was repeated, but now for a period of 2 hours.

Comparative experiment F

Comparative experiment B was repeated, with 3.5 wt.% Mg (II) being additionally applied.

Comparative experiment G

It was attempted to duplicate example III of GB-A-2001317 (NL-A-78.07199); under the conditions specified therein it appeared to be impossible, at atmospheric pressure, to raise the temperature above 150°C, so that no formation of phenol occurred. By adapting the xylene content to 10 wt.% relative to the reactor mass it was possible to raise the temperature to 215°C. Further conditions of this comparative experiment G are analogous to those of comparative experiment D.
The results of comparative experiment D-G are given in table 3.

Table 3

| Results | | | | | | |
|---|---|---|---|---|---|---|
| No. | time (hrs) | temp. (°C) | pressure ($10^5$ Pa) | ΣF (mmole) | Σm-product (mmole) | $S_f$ % |
| D | 1.0 | 218 | 1.0 | 46 | 35 | 56.8 |
| E | 2.0 | 218 | 1.0 | 47 | 37 | 56.0 |
| F | 2.0 | 218 | 1.0 | 100 | 34 | 74.6 |
| G | 1.0 | 218 | 1.0 | 100 | 102 | 49.5 |

As appears from these tests, separate performance of the reduction and hydrolysis steps, as specified in GB-A-2001317, results first of all in the formation of o-, m- and phenylbenzoates, which are the coupling products linking two arylcarboxylic acids with each other. The o- and p-products are evidently converted into the corresponding phenols in the subsequent hydrolysis. The resulting m-products, constituting 25-50% of the resulting products, cannot be converted into the corresponding phenols under the stated process conditions.

## Claims

**1.** Process for the preparation of a phenol by an oxidative decarboxylation in the liquid phase of a

corresponding arylcarboxylic acid in the presence of a Cu(I)-containing catalyst, characterized in that an oxidative decarboxylation is carried out at a temperature between 191-270 °C, in the presence of water, with such an amount of oxygen being added that so much copper remains present as Cu(I) that a selectivity to phenol is obtained of more than 92% measured at 1 bar, and phenol is separated off.

**2.** Process according to claim 1, characterized in that the oxidative decarboxylation is carried out at 210-250 °C.

**3.** Process according to any one of the claims 1-2, characterized in that during the oxidation such an amount of oxygen is supplied that 0.15 wt.% copper remains present as Cu(I).

**4.** Process for the preparation of a phenol by an oxidative decarboxylation in the liquid phase of a corresponding arylcarboxylic acid in the presence of a Cu(I)-containing catalyst, characterized in that the following process steps are performed:
a) oxidation of the catalyst at a temperature of 191-240 °C for at most 20 minutes, with such an amount of oxygen being added that at least 0.04 wt.% copper remains present as Cu(I);
b) reaction of the oxidized catalyst of step a) and formation of the phenol in the absence of oxygen, in the presence of water, at a temperature of 210-270 °C;
c) separation of phenol and recycling of the (reduced) catalyst to step a).

**5.** Process according to any one of the claims 1-4, characterized in that the oxidation of the catalyst is carried out with a gas containing 1-15% oxygen.

**6.** Process according to any one of the claims 4-5, characterized in that the oxidation is carried out in 3-10 minutes.

**7.** Process according to any one of the claims 1-6, characterized in that the Cu-containing catalyst additionally contains a cocatalyst.

**8.** Process according to any one of the claims 4-7, characterized in that step a) is carried out at a temperature of 210-240 °C.

**9.** Process according to any one of the claims 1-8, characterized in that the copper concentration (as metal and relative to the reaction mixture) amounts to 1-10 wt.%.

**10.** Process according to any one of the claims 4-9, characterized in that step b) is carried out with a virtually equimolar amount of water relative to the amount of Cu(II).

**11.** Process according to any one of the claims 4-10, characterized in that step a) and step b) are carried out at approximately the same temperature.

**12.** Process according to any one of the claims 4-11, characterized in that step b) is carried out at a temperature of 220-250 °C.

European Patent Office

# EUROPEAN SEARCH REPORT

Application Number

EP 92 20 1703

| Category | Citation of document with indication, where appropriate, of relevant passages | Relevant to claim | CLASSIFICATION OF THE APPLICATION (Int. Cl.5) |
|---|---|---|---|
| X | US-A-4 405 823 (MAKI et al.) * Column 2, line 57 - column 3, line 15; column 3, line 62 - column 4, line 5; column 5, lines 9-42; claims 1,6,7 * --- | 1-4,6-9,11,12 | C 07 C 37/56<br>C 07 C 39/04 |
| A | US-A-2 766 294 (W.G. TOLAND) * Column 2, lines 6-37; column 3, lines 25-35; column 4, line 65 - column 5, line 8; column 6, lines 16-32; claims 1-3 * ----- | 1,2,7 | |

**TECHNICAL FIELDS SEARCHED (Int. Cl.5)**

C 07 C 37/00
C 07 C 39/00

The present search report has been drawn up for all claims

| Place of search | Date of completion of the search | Examiner |
|---|---|---|
| THE HAGUE | 11-09-1992 | KLAG M.J. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document